# EUROPÄISCHE PATENTSCHRIFT

(11) **EP 1 007 968 B1**
(45) Veröffentlichungstag und Bekanntmachung des Hinweises auf die Patenterteilung: **12.12.2001**
(21) Anmeldenummer: 98947468.9
(22) Anmeldetag: 28.08.1998
(51) Int. Cl.: G01N 33/53

(54) **VERFAHREN UND KIT ZUR IDENTIFIZIERUNG VON WECHSELWIRKUNGEN ZWISCHEN PROTEINEN BZW. PEPTIDEN**
METHOD AND KIT FOR IDENTIFYING INTERACTIONS BETWEEN PROTEINS OR PEPTIDES
PROCEDE ET KIT POUR IDENTIFIER DES INTERACTIONS ENTRE DES PROTEINES OU DES PEPTIDES

(30) Priorität: 28.08.1997 DE 19737562
(43) Veröffentlichungstag der Anmeldung: 14.06.2000
(73) Patentinhaber: Otogene Aktiengesellschaft, 72070 Tübingen (DE)
(72) Erfinder: PAYSAN, Jacques, D-70599 Stuttgart (DE); HERLITZE, Stefan, D-72074 Tübingen (DE); ANTZ, Christof, D-72070 Tübingen (DE); RUPPERSBERG, Peter, D-72072 Tübingen (DE)
(74) Vertreter: Patentanwälte Ruff, Wilhelm, Beier, Dauster & Partner
(86) Internationale Anmeldenummer: EP9805458
(87) Internationale Veröffentlichungsnummer: WO9912033

(56) Entgegenhaltungen:
- EP-A- 0 242 527
- EP-A- 0 870 829
- WO-A-91/01305
- WO-A-91/09312
- WO-A-91/12530
- WO-A-97/20078
- WO-A-97/27212
- WO-A-98/48278
- US-A- 5 503 977
- US-A- 5 563 039
- US-A- 5 616 475

## Beschreibung

Die vorliegende Erfindung betrifft ein Verfahren und einen Kit zur Identifizierung oder Bestimmung von Wechselwirkungen zwischen Proteinen bzw. Peptiden mittels Fluoreszenz-Resonanz-Energie-Transfer.

Die Identifizierung und Analyse von Wechselwirkungen zwischen verschiedenen Proteinen bzw. Peptiden oder Fragmenten davon stellt ein wichtiges Problem der biomedizinischen Forschung und Biotechnologie dar. Ende der 80iger Jahre wurde deshalb ein System entwickelt, das unter der Bezeichnung "Yeast Two-Hybrid-System" in der Forschung große Bedeutung erlangt hat (Fields et al., Nature 340, S. 245-247 (1989)). Dieses System basiert auf der Entdeckung, daß zelluläre Transkriptionsaktivatoren, wie z.B. GAL4 oder lexA aus Hefe, in zwei unabhängige Funktionsdomänen zerlegt werden können. Beide Domänen sind normalerweise Bestandteil eines Proteins im Zellkern der Hefezelle, welches an bestimmte aktivierende Sequenzen verschiedener Zielgene bindet und deren Transkription reguliert. Dabei bindet die eine Domäne, die DNA-Bindungsdomäne (BD), spezifisch an eine bestimmte DNA-Zielsequenz (upstream activating sequence) in der Nähe des Zielgenpromotors. Die andere Domäne, die Aktivierungsdomäne (AD), erhöht die Transkriptionsrate des Zielgens durch Wechselwirkung mit dem Transkriptionsinitiationskomplex, der am Promotor des Zielgens gebunden ist. Im "Yeast Two-Hybrid-System" wird diese Struktur von Transkriptionsfaktoren in veränderter Form ausgenutzt. Die DNA-Bindungsdomäne (BD) von GAL4 oder lexA wird dort als Fusionsprotein mit einem "Köderprotein oder -peptid" in Hefezellen exprimiert. Dieses Fusionsprotein besitzt außerdem ein Kernlokalisierungssignal, durch welches es in den Zellkern der Hefe transportiert wird. Das Köder-Fusions-protein bindet dort an eine Zielsequenz (UAS), die sich in dem verwendeten Hefestamm in der Nähe der Promotoren von zwei Reportergenen (z.B. auxotropher Marker (HIS3) und enzymatischer Marker (lacZ)) befindet. Dadurch entsteht eine Konstellation, in der das Köderprotein oder -peptid in direkter räumlicher Nähe des Reportergenpromotors exponiert wird. In derselben Hefezelle wird nun zusätzlich ein zweites Fusionsprotein exprimiert. Dieses besteht aus der Aktivierungsdomäne (AD) von GAL4 oder lexA und einem Beuteprotein -oder peptid. Es besitzt ebenfalls ein Kernlokalisierungssignal. Das Beute-Fusionsprotein wird also auch in den Zellkern der Hefe transportiert. Falls nun das Beuteprotein und das an der UAS exponierte Köderprotein eine physikalische Wechselwirkung miteinander eingehen, dann erhöht sich die statistische Wahrscheinlichkeit, daß die Aktivierungsdomäne sich in der Nähe des Reportergenpromotors aufhält. Dadurch kommt es zu einer Steigerung der Transkription der Reportergene, deren Ausmaß proportional zur Stärke der Wechselwirkung zwischen Köder- und Beuteprotein ist. Das "Yeast Two-Hybrid-System" kann sowohl zur quantitativen Analyse bekannter Beute/Köderpaare als auch zur Identifizierung unbekannter Beuteproteine oder -peptide verwendet werden. Dabei kommen als Beuteproteine z.B. eine cDNA-Bibliothek oder auch eine kombinatorische Peptidbibliothek in Frage.

Trotz der oben beschriebenen vielseitigen Anwendungsbereiche weist das "Yeast Two-Hybrid-System" Einschränkungen aufgrund des transkriptionsabhängigen Detektionssystems auf. Diese treten beispielsweise dann auf, wenn das Beute- und/oder Köderprotein selbst Lokalisierungssignale enthalten. Diese störenden Lokalisierungssignale sind z.B. hydrophobe Transmembrandomänen, wie sie in vielen Membranproteinen auftreten. Sie führen zu einem Transport des Fusionsproteins in die Zellmembran, während das Kernlokalisierungssignal des Fusionsproteins ignoriert wird. Wechselwirkungen mit Proteinen, die solche Transmembrandomänen aufweisen, können deshalb nicht detektiert werden. Ein weiteres Problem besteht beim Screening zur Analyse von cDNA-Bibliotheken oder kombinatorischen Peptid-Bibliotheken, die nur in begrenztem Umfang durchzuführen sind. Letztere stellen aufgrund ihrer Komplexizität (z.B. mehr als 10 Billionen mögliche Varianten für ein Zehnerpeptid) ein effizientes Screening vor unlösbare Probleme. Die Analyse möglicher Wechselwirkungspartner erfolgt dabei bisher dadurch, daß maximal 50.000 transformierte Hefezellen pro Agarplatte ausplattiert werden und zunächst 3-7 Tage im Brutschrank inkubiert werden. Dies bedeutet, daß ein typischer Test in der Größenordnung von 5 Millionen möglichen Fusionsproteinpaaren bereits die Verwendung von 100 Agarplatten erfordert, die durchgetestet werden müssen. So ist das "Yeast Two-Hybrid-System" für diese Screening-Vorgänge mit einem hohen Aufwand für Material, Zeit, Arbeitskraft und Kulturraum verbunden. Außerdem ist kein automatisiertes Verfahren zur Analyse bekannt, das den hohen Arbeitsaufwand für Laborkräfte reduzieren und standardisieren würde.

In der internationalen Patentanmeldung WO 97/27212 wird ein Screening-Verfahren vorgestellt, mit welchem Peptidsequenzen identifiziert werden, die den Phänotyp einer Zelle meßbar verändern. Das Verfahren beruht auf der Messung der Phänotypänderung nach Expression des Peptids in der Zelle. Hier werden zunächst keine Wechselwirkungen von Peptiden oder Proteinen untersucht. Erst in einem zweiten Schritt können die identifizierten wirksamen Peptidsequenzen näher untersucht werden. Hierzu sind verschiedene bekannte Methoden alternativ einsetzbar, beispielsweise kann die Abwesenheit von normalen zellulären Funktionen gemessen werden, ein Two-hybrid-System in Säugetierzellen eingesetzt oder ein Fluoreszenz-Resonanz-Energie-Transfersystem verwendet werden.

In der Literatur sind bereits einige Testsysteme zur Untersuchung bekannter Wechselwirkungspartner beschrieben, die den Fluoreszenz-Resonanz-Energietransfer ausnutzen. Beispielsweise wird in der internationalen Patentanmeldung WO 91/12530 ein Fluoreszenztest zur Untersuchung von Wechselwirkungen bestimmter Bindungspartner, vor allem im Hinblick auf Immunoassays, beschrieben. Die europäische Patentanmeldung EP 0242527 beschreibt ein Nachweisverfahren für wechselwirkende Analyten unter Verwendung des Energie-Transfersystems.

Die Aufgabe der vorliegenden Erfindung besteht deshalb darin, ein Verfahren und einen entsprechenden Kit zur Identifizierung von Wechselwirkungen zwischen Proteinen bzw. Peptiden bereitzustellen, das die obigen Nachteile mindestens teilweise vermeidet und dabei insbesondere automatisiert durchgeführt werden kann.

Diese Aufgabe wird durch die Gegenstände der Patentansprüche gelöst. Der Wortlaut sämtlicher Ansprüche wird hiermit durch Bezugnahme zum Inhalt dieser Beschreibung gemacht.

Insbesondere wird diese Aufgabe durch ein Verfahren gelöst, in dem mindestens zwei Proteine oder Peptide an unterschiedliche fluoreszierende Komponenten gekoppelt sind, wobei die Absorptions- und Emissionsspektren der fluoreszierenden Komponenten derart überlappen, daß Fluoreszenz-Resonanz-Energie-Transfer (FRET) möglich ist und die Komponenten durch eine Wechselwirkung zwischen den Proteinen bzw. Peptiden so zusammengebracht werden, daß FRET auftritt und gemessen wird. Hierbei liegt die genetische Information für mindestens zwei Fusionspeptide bzw. -proteine aufweisend einen Peptid- bzw. Proteinanteil und je ein unterschiedliches fluoreszierendes Protein bzw. Peptid in einem Expressionssystem vor, welches in eine Wirtszelle eingebracht wird, wobei die Emissions- und Absorptionsspektren der fluoreszierenden Proteine bzw. Peptide derart überlappen, daß Fluoreszenz-Resonanz-Energie-Transfer (FRET) auftritt und in der Wirtszelle gemessen wird.

Im Rahmen der vorliegenden Erfindung sollen die Begriffe "Protein" und "Peptid" gegeneinander austauschbar sein und eine Aminosäuren-Aneinanderreihung jeglicher Länge und Komplexizität beinhalten, d.h. Dipeptide, Oligopeptide, Polypeptide, vollständige Proteine, Fragmente davon, Antikörper, Domänen, Epitope usw. Insbesondere kann es sich um kombinatorische Peptidbibliotheken oder die Expressionsprodukte von cDNA-Bibliotheken handeln. Insgesamt unterliegen die auf Wechselwirkungen zu untersuchenden Proteine/Peptide keinerlei Beschränkungen und sind frei wählbar.

Insbesondere soll erfindungsgemäß unter einer fluoreszierenden Komponente ein Material bzw. sollen Partikel verstanden werden, die an der Oberfläche eine fluoreszierende Markierung aufweisen. Dies können Latex-Partikel sein oder Matrices, wie sie für die automatische Proteinsynthese üblich sind.

Erfindungsgemäß soll unter Kopplung eine mehr oder weniger feste Bindung der fluoreszierenden Komponente an das bzw. die Proteine oder Peptide verstanden werden. Diese Bindung kann mehr adsorptiver Natur sein oder es kann sich auf der anderen Seite auch um eine kovalente Bindung handeln. In einer bevorzugten Ausführungsform können das Protein bzw. Peptid und die fluoreszierende Komponente in Form eines Fusionsproteins bzw. -peptids vorliegen. Die Kopplung kann auch mittels eines Linkers stattfinden. Dieser umfaßt Verbindungen jeglicher Art, die zur Verknüpfung zweier Moleküle geeignet sind.

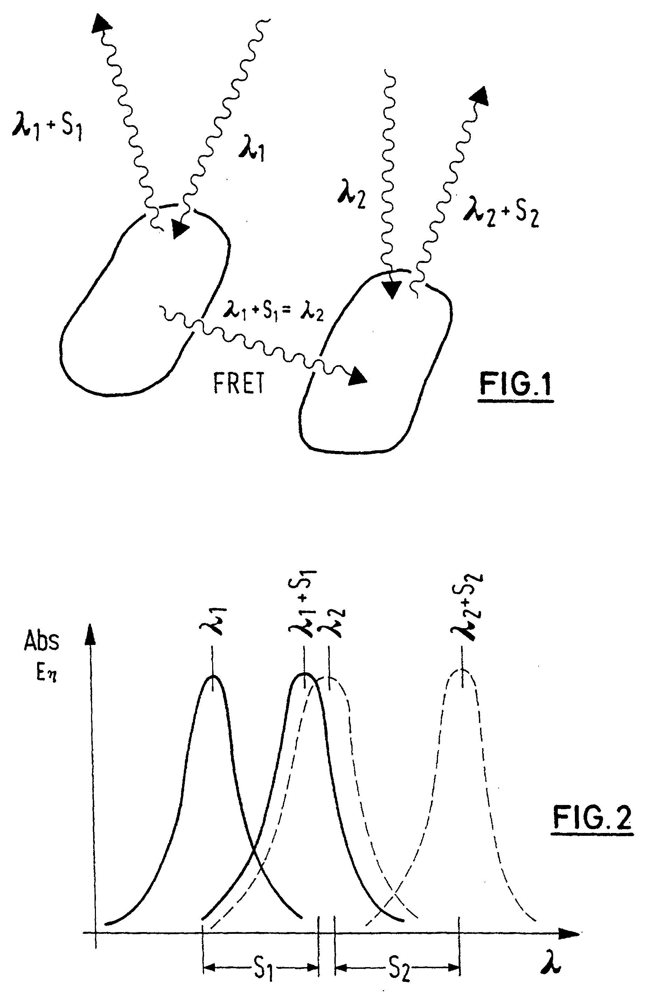

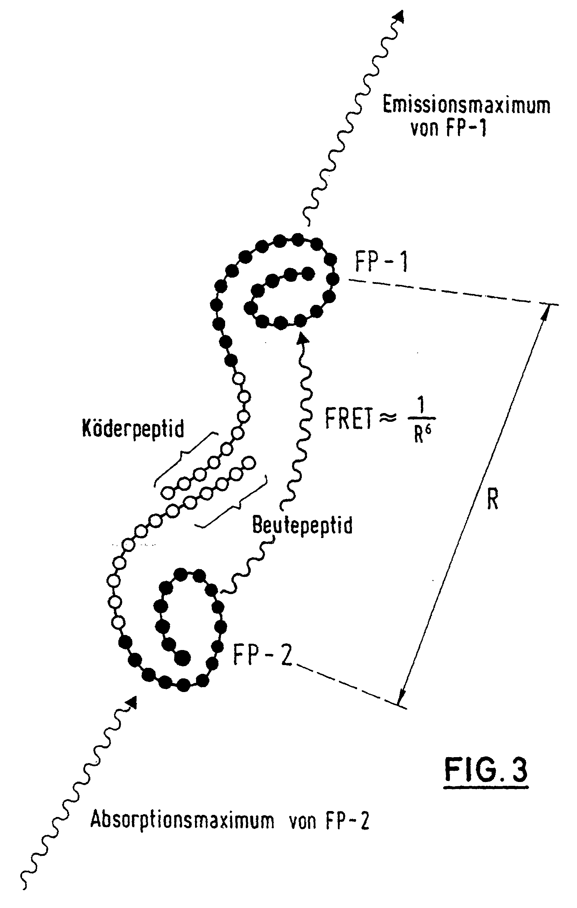

Das erfindungsgemäße Verfahren beruht u.a. auf dem bekannten Phänomen des Fluoreszenz-Resonanz-Energietransfers (FRET), der in Fig. 1 schematisch gezeigt ist. Ein fluoreszierendes Molekül absorbiert Photonen mit einer charakteristischen Wellenlänge und setzt die so aufgenommene Energie innerhalb kürzester Zeit durch Emission von Photonen wieder frei, was eine meßbare Fluoreszenz hervorruft. Da während dieses Prozesses ein gewisser Energieverlust durch Wärmeentwicklung auftritt, hat das emittierte Photon einen charakteristisch verminderten Energiegehalt und somit eine veränderte Wellenlänge gegenüber dem zuvor absorbierten Photon (Stoke'sche Verschiebung). Beide Parameter, nämlich Absorptionswellenlänge und Stoke'sche Verschiebung (und davon abhängig auch die Emissionswellenlänge) sind charakteristische Parameter eines jeden fluoreszierenden Moleküls und hängen von dessen Beschaffenheit ab. Ein Fluoreszenz-Resonanz-Energie-Transfer läßt sich nun messen, wenn zwei fluoreszierende Moleküle in einer Mischung miteinander in Wechselwirkung treten und die Absorptionswellenlänge des einen mit der Emissionswellenlänge des anderen überlappt. In diesem Zusammenhang sei auf die Fig. 2 und 3 hingewiesen. Wird durch Licht der Wellenlänge λ1 das fluoreszierende Molekül F1 angeregt, so emittiert F1 Licht der Wellenlänge λ1 + s1, wobei sl der Betrag der Stoke'schen Verschiebung von F1 ist. Das zweite fluoreszierende Molekül F2 kann dieses Licht absorbieren, wenn sein Absorptionsmaximum λ2 mit λ1 + s1 überlappt oder im Idealfall übereinstimmt. Das so durch λ1 +s1 angeregte F2 wird seinerseits die Wellenlänge λ2 + s2 emittieren. Da diese Wellenlänge nicht charakteristisch für F1 ist, kann man auf einen erfolgten Energietransfer zwischen F1 und F2 schließen. Die Häufigkeit solcher Transferereignisse ist statistisch. Die Effizienz des Energietransfers ist proportional zu r⁶, wobei r der mittlere Abstand zwischen den beiden fluoreszierenden Molekülen ist. Jede Wechselwirkung zwischen den beiden Molekülen und jede Erhöhung ihrer Affinität zueinander, wird deshalb zu einem dramatischen Anstieg in der Häufigkeit von Energietransferereignissen und damit zu einer meßbaren Erhöhung des FRET führen. FRET kann somit als Indikator für die Affinität zwischen fluoreszierenden Molekülen herangezogen werden. Die Detektion von FRET kann durch bekannte fluorometrische Methoden, z.B. fluoreszenzaktivierte Zellsortierung (FACS) oder Fluoreszenzmikroskopie, erfolgen.

In einer bevorzugten Ausführungsform der vorliegenden Erfindung wird die genetische Information für zwei Fusionsproteine (im nachfolgenden A und B genannt) gemeinsam in einer Wirtszelle, bevorzugt eukaryotischen Zelle, exprimiert und Änderungen im Fluoreszenzspektrum dieser Zelle als Kriterium für eine mögliche Wechselwirkung zwischen den Fusionsproteinen herangezogen.

Dabei besteht das Fusionsprotein A aus einem Protein bzw. Peptid (auch Köderprotein/-peptid genannt), für die ein unbekannter Wechselwirkungspartner identifiziert oder ein bekannter Wechselwirkungspartner charakterisiert werden soll.

Das Köderprotein ist mit einem fluoreszierenden Protein (FP-A) gekoppelt. Das Fusionsprotein B besteht aus einem bekannten oder unbekannten Protein bzw. Peptid (auch Beuteprotein/-peptid genannt), dessen physikalische Bindungsfähigkeit charakterisiert werden soll. Das Beuteprotein ist an ein anderes fluoreszierendes Protein (FP-B) gekoppelt.

Die fluoreszierenden Proteine bzw. Peptide FP-A und FP-B zeichnen sich dadurch aus, daß sich die Emissions- und Absorptionspektren von FP-A und FP-B derart überlappen, daß Fluoreszenz-Resonanz-Energie-Transfer zwischen den beiden Molekülen möglich wird. Dafür in Frage kommt das Green Fluorescent Protein aus Aequorea victoria (GFP) und verschiedene seiner Mutationsvarianten. Die für diese fluoreszierenden Proteine kodierenden cDNAs sind käuflich erhältlich (z.B. Fa. Clontech, Palo Alto, USA). Bevorzugt wird für FP-A das Blue Fluorescent Protein (BFP) und für FP-B das Green Fluorescent Protein (GFP) eingesetzt. BFP kann mit einer Wellenlänge von 380 nm angeregt werden und emittiert maximal bei 440 nm. GFP hingegen wird maximal bei 488 nm angeregt und emittiert bei 507 nm. Die Emission bei 507 nm wird signifikant erhöht werden, wenn es durch Protein-Protein-Wechselwirkungen zum Fluoreszenzenergietransfer zwischen FP-A (BFP) und FP-B (GFP) kommt. Erfindungsgemäß ist es natürlich auch möglich, für FP-A das Green Fluorescent (GFP) und für FP-B das Blue Fluorescent Protein (BFP) einzusetzen.

Die Fusion der genetischen Information des fluoreszierenden Proteins FP-A mit dem Köderprotein und des fluoreszierenden Proteins FP-B mit dem Beuteprotein erfolgt über molekularbiologische Standardmethoden, die dem Fachmann bestens bekannt sind. Dazu wird die kodierende DNA-Sequenz eines Köderproteins mit der kodierenden DNA-Sequenz des fluoreszierenden Proteins FP-A z.B. mittels enzymatischer Ligation verbunden und gemäß Standardmethoden in einen geeigneten Expressionsvektor kloniert. Außerdem wird die kodierende DNA-Sequenz eines Beuteproteins mit der kodierenden DNA-Sequenz des fluoreszierenden Proteins FP-B z.B. mittels enzymatischer Ligation verbunden und gemäß Standardmethoden in einen Expressionsvektor kloniert. Es kann hier jeweils auch eine beliebig komplexe Population von Köder- und Beuteproteinen verwendet werden, um eine Bibliothek möglicher Wechselwirkungspartner zu erstellen. Unter genetischer Information sowie kodierender DNA-Sequenz soll erfindungsgemäß cDNA oder genomische DNA, bevorzugt cDNA, verstanden werden.

Als Expressionsvektor eignen sich die dem Fachmann bekannten üblichen Vektoren. Allerdings sollten diese eine getrennte Selektion von "Köderprotein-Vektor" und "Beuteprotein-Vektor" in den Wirtszellen ermöglichen, z.B. durch unterschiedliche Antibiotikaresistenzgene (z.B. Ampicillin, Kanamycin, Chloramphenicol, Streptomycin, Tetracykline, Sulfonamide) oder unterschiedliche auxotrophe Marker (z.B. LEU2, HIS3 oder TRP1). Es ist jedoch auch möglich, beide Fusionsproteine gemeinsam in einen bicistronischen Expressionsvektor zu kombinieren. Geeignete Expressionsvektoren sind für die Expression in E. coli z.B. pGEMEX, pUC-Derivate, pGEX-2T, pET3b, pQE8 oder pQE42, für die Expression in Hefe pY100, Ycpad1, pGBT9 oder pGAD424, für die Expression in tierischen Zellen pKCR, pEFBOS, cDM8 und pCEV4. Für die Expression in Insektenzellen eignet sich besonders der Baculovirus-Expressionsvektor pAcSGNt-A.

Ferner kennt der Fachmann Verfahren und Wirtszellen, um den Expressionsvektor und das durch ihn kodierte Fusionsprotein zu exprimieren. Beispiele solcher Zellen umfassen die E. coli Stämme HB101, DH1, x1776, JM101, Jm109, B121 und SG13009, den Hefestamm Saccharomyces cerevisiae, Schizosaccharomyces pombe, Y 190, CG1945, EGY48 oder HF7, die tierischen Zellen L, 3T3, FM3A, CHO, COS, Vero und Hela sowie die Insektenzellen Sf9. Geeignete Zellen, bevorzugt Hefezellen, werden mit dem bzw. den Expressionsvektoren transformiert (sequentiell oder synchron) und bevorzugt unter doppelter Selektion vermehrt.

Anschließend wird die Fluoreszenz der Zellen beim Absorptionmaximum eines der beiden fluoreszierenden Proteine (bevorzugt beim Maximum von FP-A) angeregt, beim Emissionsmaximum des anderen fluoreszierenden Proteins (bevorzugt von FP-B) gemessen und das Meßergebnis als Selektionskriterium bei der Isolierung und Klonierung von Zellen herangezogen, die ein potentielles Wechselwirkungspaar beinhalten. Diese Messung wird zuvor durch Zellen kalibriert, in denen FP-A und FP-B in verschiedene Vektoren kloniert exprimiert werden, ohne jedoch mit einer weiteren Proteindomäne fusioniert zu sein (Negativkontrolle). Als Positivkontrolle kann ein Fusionsprotein dienen, in dem FP-A und FP-B (ohne Köder- oder Beuteprotein) in der oben beschriebenen Weise aneinander gekoppelt werden und somit eine maximale räumliche Nähe zwischen FP-A und FP-B gegeben ist.

Die Anregung der Fluoreszenz erfolgt bevorzugt mittels Laser. Die Messung auf FRET erfolgt unter Verwendung geeigneter Filterkombinationen, die vom Fachmann in Abhängigkeit von den Absorptions- und Emissionsmaxima der verwendeten fluoreszierenden Proteine ausgewählt werden können. Entsprechend der Grundlagen von FRET muß jede Wechselwirkung zwischen den Fusionsproteinen zu einem Anstieg von FRET führen, der nach geeigneter Kalibrierung des Systems detektiert werden kann. Diese Detektion erfolgt für die Untersuchung bekannter Köder/Beute-Paare bevorzugt fluoreszenzmikroskopisch. Für das Screening von cDNA-Bibliotheken oder kombinatorischen Peptidbibliotheken erfolgt die Messung bevorzugt mittels fluoreszenzgesteuerter Zellsortierung (FACS).

Zur weiteren Identifikation der durch FRET ermittelten Wechselwirkungspartner werden diese auf Agarplatten ausplattiert und aus den individuellen Kolonien Plasmid-DNA isoliert. Diese können dann mittels Standardmethoden sequenziert werden.

Das erfindungsgemäße Verfahren hat beispielsweise folgende bevorzugte Anwendungen:
- Ein bekanntes Paar möglicher Wechselwirkungspartner wird gemeinsam in einer Wirtszelle exprimiert und deren resultierendes Fluoreszenzspektrum mit dem von Negativ- und Positivkontrollen bzw. anderen Wechselwirkungspartnern verglichen;
- Ein Köder-Fusions-Protein wird mit einer beliebig komplexen Mischung von Beute-Fusions-Proteinen (gemeinsam oder sequentiell) in geeignete Wirtszellen transformiert bzw. transfiziert und zwar möglichst so, daß jede Zelle nur ein definiertes Paar von möglichen Wechselwirkungspartnern trägt bzw. eine kleine Gruppe solcher Paare. Aus der so entstehenden Wirtszellpopulationen werden dann die Zellen isoliert, die einen signifikanten FRET aufweisen und somit ein potentielles Wechselwirkungspaar exprimieren. Durch Isolierung der in diesen Zellen enthaltenen Expressionsvektoren kann dann leicht die Sequenz der beteiligten Wechselwirkungspartner bestimmt werden. Die Mischung der Beuteproteine könnte z.B. aus cDNA-Bibliotheken oder aus kombinatorischen Peptidbibliotheken bestehen.
   Auch das Köderprotein könnte durch eine Population verschiedener Proteine vertreten sein. Dadurch ist es beispielsweise möglich, eine Interaktionmatrix zu bestimmen, z.B. indem eine cDNA-Bibliothek auf mögliche Wechselwirkungspaare zwischen unbekannten Proteinen durchsucht wird;
- Ein bekanntes Paar von wechselwirkenden Fusionsproteinen wird gemeinsam in Wirtszellen exprimiert und der Fluoreszenztransfer zwischen den beiden Fusionsproteinen ermittelt. Dann wird ein drittes Protein oder Peptid in denselben Wirtszellen exprimiert und dessen Einfluß auf den FRET zum Analysekriterium gemacht. Auf diese Weise können Proteine oder Peptide ermittelt werden, die eine existierende Wechselwirkung zwischen bekannten Wechselwirkungspartnern stören oder aber verstärken.

Die Vorteile des erfindungsgemäßen Verfahrens liegen darin, daß es im Gegensatz zum "Yeast Two-Hybrid-System" unabhängig von transkriptionsregulierenden Mechanismen ist und die Lokalisierung der Fusionsproteine im Kern unerheblich ist. So können beispielsweise auch intakte Membranproteine als Köderprotein verwendet werden.

Zusätzlich besteht die Möglichkeit einer automatisierten Zellsortierung. Dadurch kann eine hohe Screeningkomplexizität erzielt werden. Durch Kombination einer pharmakologisch interessanten Zielsequenz mit einer kombinatorischen Peptidbibliothek können mehrere Millionen Peptidliganden pro Zeiteinheit gescreent werden. Das erfindungsgemäße Verfahren zeichnet sich dabei gegenüber bekannten Verfahren, mit denen dies möglicherweise gar nicht durchführbar gewesen wäre, durch eine enorme Zeit- und Arbeitsersparnis aus.

Der erfindungsgemäße Kit (Test-Kit) ist in Anspruch 7 beschrieben. Bevorzugte Ausführungsformen dieses Kits gehen aus den abhängigen Ansprüchen 8 bis 15 hervor. Auch der Wortlaut dieser Ansprüche 7 bis 15 wird hiermit durch Bezugnahme zum Inhalt dieser Beschreibung gemacht. Auf die bisherigen Teile der Beschreibung, die direkt oder indirekt mit den Merkmalen der Ansprüche 7 bis 15 im Zusammenhang stehen, wird ausdrücklich verwiesen und Bezug genommen.

Der beanspruchte Kit dient dazu, das beanspruchte Verfahren in besonders einfacher Weise durchzuführen. In dem Kit ist dabei vorzugsweise in einem geeigneten Behältnis ein erster Vektor vorhanden, der die wirtszelloptimierte kodierende Sequenz eines FRET-Donors (unter der Kontrolle eines geeigneten Promotors) trägt, und bei dem eine Klonierungsmöglichkeit für das Einbringen einer kodierenden Sequenz, die für ein erstes Testprotein/Testpeptid, (d.h. ein Köder- oder Beuteprotein oder -peptid) kodiert, vorgesehen ist.
Weiter ist ein zugehöriger zweiter Vektor vorhanden, mit wirtszelloptimierter kodierender Sequenz für den zugehörigen FRET-Akzeptor und Klonierungsmöglichkeit für die kodierende Sequenz eines zweiten Testproteins/Testpeptids (entsprechender Wechselwirkungspartner für das auszubildende Köder-Beute-Paar).

Die kodierenden Sequenzen für die Testproteine/Testpeptide im ersten und/oder zweiten Vektor können bereits vorhanden sein. Vorzugsweise können auch integrierte cDNA-Bibliotheken oder kombinatorische Oligonukleotid-Bibliotheken enthalten sein. In solchen Fällen wird beispielsweise eine cDNA-Bibliothek (z.B. aus humanem Gehirn) mit einem der FRET-Partner in einem ersten Vektor fusioniert vorliegen und ein zweiter Vektor, in den der Änwender ein eigenes Protein/Peptid, z.B. als Köder, mit dem anderen FRET-Partner klonieren kann.

Die Erfindung wird weiter anhand der Figuren beschrieben, welche zeigen:
- Fig. 1:: Schematische Darstellung von Fluoreszenz-Resonanz-Energietransfer (FRET)
- Fig. 2:: Schematische Darstellung der Energieverschiebung
- Fig. 3:: Schematische Darstellung von FRET anhand der Wechselwirkung von Köderprotein mit Beuteprotein
- Fig. 4:: Genkarte des Expresssionsvektors pGBT9
- Fig. 5:: Genkarte des Expressionsvektors pGAD424

Die Erfindung wird weiter anhand der nachfolgenden Ausführungsbeispiele beschrieben.

### BEISPIEL 1:

Zunächst wird ein geeignetes Vektorensystem konstruiert. Hierzu werden der Einfachheit halber die Plasmide pGBT9 (#K-1605-A), pGAD424 (#K1605-B) der Firma Clontech (Palo Alto, US) sowie die Plasmide pRSET B-P4-3 und pRSET B-S65T (Heim und Tsien, Current Biology 1996, 6: 178-182) benutzt.

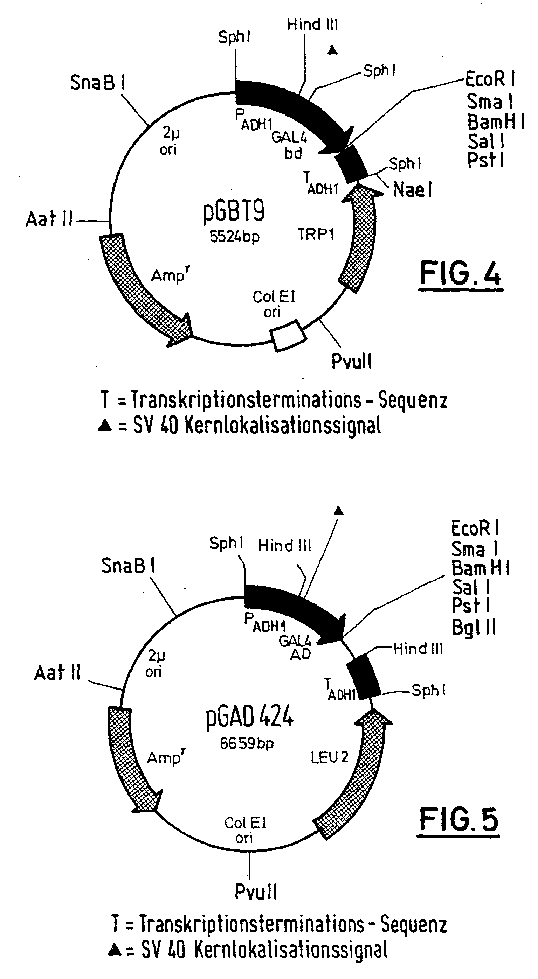

pGBT9 ist ein möglicher "Köderproteinvektor" des Yeast Two Hybridsystems, das von Clontech vertrieben wird. Der Vektor enthält ein Ampicillinresistenzgen zur Selektion in Bakterien, ein TRP1 Gen zur auxotrophen Selektion in Hefe, sowie eine Expressionskassette für die DNA-bindende Domäne des GAL4 Transkriptionsfaktors (Fig. 4, Clontech Matchmaker "GAL4 Two-Hybrid Vektors Handbook, #PT3062-1) unter der Kontrolle eines Alkoholdehydrogenasepromotors (ADH1) zur Expression in Hefe. Die GAL4-DNA bindende Domäne ist ein essentieller Bestandteil des Yeast Two-Hybrid Systems.

Für den erfindungsgemäßen Zweck wird die GAL4 DNA-bindende Domäne mitsamt ihrem Kernlokalisationssignal entfernt, indem sie über einen HinDIII/EcoRI-Restriktionsverdau ausgeschnitten wird.

pGAD424 ist ein möglicher "Beuteproteinvektor" des Yeast Two-Hybrid Systems (Fig. 5). Dieser Vektor ist ähnlich aufgebaut wie pGBT9, nur daß er statt des TRP1-Markers ein LEU2 Gen für die Selektion in Hefe enthält und statt der GAL4 DNA-bindenden Domäne die GAL4 Aktivierungsdomäne enthält.

Für den erfindungsgemäßen Zweck wird auch aus diesem Vektor die GAL4 Aktivierungsdomäne durch einen HinDIII/EcoRI Verdau entfernt. Dazu werden zunächst weitere im Vektor befindliche HinDIII Schnittstellen über Partialverdau, Auffüllen der Schnittstelle mit Pfu-Polymerase und anschließender "blunt-end-Ligation" entfernt.

Geeignete kodierende Sequenzen der GFP-Varianten S65T und P4-3 (Heim und Tsien, Current Biology 6: 178-182 (1996) werden nun durch PCR amplizifiert und an ihren 5' und 3'-Enden mit Hin DIII bzw. EcoRI-Schnittstellen versehen. Dazu werden die Oligonucleotidprimer GFP-EcoRI-3'(5'-CGGGAATTCTTTGTATAGTTCATCCAT-3'), sowie GFP-HinDIII-5'-TCCAAGCTTATGAGTAAAGGAGAAGAACTT-3') (Carl Roth GmbH, Karlsruhe) verwendet. Da die kodierenden Sequenzen von P4-3 und S65T in ihren 5' und 3'-terminalen Regionen identisch sind, kann das gleiche Primerpaar für beide Reaktionen verwendet werden.

Besonders geeignet sind kodierende Sequenzen mit einer auf Hefe abgestimmten Nutzung des Kodons, z.B. Sequenzen, die von yEGFB abgeleitet sind (Microbiology 143: 303 - 311 (1997)).

Die so gewonnenen kodierenden Sequenzen von P4-3 und S65T werden nun über eine Gelelektrophorese aufgereinigt und durch eine Ligasereaktion in die wie oben beschrieben vorbereiteten Vektoren pGBT9 und pGAD424 kloniert. Die resultierenden Konstrukte pGBT-BFP und pGAD-GFP enthalten nun die kodierende Sequenz des Blau Fluoreszierenden Proteins BFP (P4-3) bzw. des Grün Fluoreszierenden Proteins GFP (S65T) unter der Kontrolle des ADH1-Promotors zur Expression in Hefe. Beiden GFP-Varianten ist außerdem der ursprüngliche Polylinker der Plasmide pGBT9 und pGAD424 nachgestellt. Sie ersetzen die für das Yeast Two-Hybrid System essentiellen Komponenten des GAL4 Transkriptionsfaktors.

In gleicher Weise und mit besserem Ergebnis eigenen sich kodierende Sequenzen der Proteine Enhanced-Cyan-Fluorescent-Protein (ECFP; Mutation T66W) und Enhanced-Yellow-Fluorescent-Protein (EYFP, Mutation T203Y), abgeleitet von Enhanced-Green-Fluorescent-Protein (Miyawaki et al., Nature **388**: 882-887, 1997; Clontech-Manual) und abgestimmt auf die Kodon-Nutzung der Hefe.

Die gewonnenen Plasmide werden nun in kompetente Zellen E.coli DH5 α (Gibco BRL) transformiert und in ampicillinhaltigem LB-Medium amplifiziert. Nach einer Plasmidpräparation durch Qiagen Kits steht somit ein geeignetes Vektorensystem zur Verfügung.

Als Beispiel für ein geeignetes Köderprotein dient die C-terminale Domäne des Purinrezeptors P2X2 (Brändle et al., FEBS Lett. 404: 294k-298 (1997)). Davon wird die in einem Vektor vorliegende cDNA verwendet. Durch eine PCR Amplifikation wird die Domäne zwischen der Transmembrandomäne TM2 und dem intrazellulären C-Terminus amplifiziert. Dazu werden die Oligonukleotidprimer P2X2-5' (5'-CGGGAATTCACGTTCATGAACAAAAAC-3') und P2X2-3' (5'TTAGGATCCTCAAAGGGCCAAACCTTTGGGGTC-3') verwendet. Das gewonnene Fragment wird dann über die Restriktionsschnittstellen Eco RI und BamHI in den Vektor pGBT-BFP kloniert und in der üblichen Weise amplifiziert.

Für eine geeignete Bibliothek von Beuteproteinen wird eine mRNA-Isolierung aus Rattenhirn und cDNA-Synthese bei einer Firma in Auftrag gegeben, die solche Serviceleistungen kommerziell anbieten, z.B. Clontech oder Stratagene. Die so erhaltene cDNA wird dann in der üblichen Weise über die EcoRI Schnittstelle in den vorbereiteten und amplifizierten Vektor pGAD-GFP kloniert, in hochkompetente E-coli Zellen (Library Efficiency DH5a, GibcoBRL) transformiert und in der für cDNA-Bibliotheken üblichen Weise amplifiziert (siehe z.B. Current Protocols in Molecular Biology, Wiley).

Um beide Konstrukte in Hefezellen zu bringen, werden kompetente Zellen des Hefestamms EGY48 (Fa. Invitrogen) hergestellt. (Dieser Hefestamm trägt zwei Mutationen, die die Gene leu2 und trp1 funktionslos machen. Diese Mutationen können durch die TRP1 und LEU2 Gene der Vektoren kompensiert werden). Dazu wird die Lithiumacetat-Methode entsprechend dem Clontech Two-Hybrid Handbuch benutzt (#PT3024-1) unter Verwendung des Yeastmaker Transformationssystems (Clontech, #K1k606-1). Die gewonnenen kompetenten Hefezellen werden zunächst mit dem Beuteplasmid pGBT-BFP-P2X2 transformiert und auf tryptophanfreien Agrarplatten ausplattiert. Die Transformanten werden dann in tryptophanfreiem Flüssigminimalmedium vermehrt und nach der Lithiumacetatmethode wieder kompetent gemacht. Es erfolgt dann eine Transformation im Bibliotheksmaßstab, wieder entsprechend dem Clontech Handbuch. Dieser Transformationsansatz wird für mehrere Stunden in einer Schüttelkultur in tryptophan- und leucinfreiem Flüssigminimalmedium amplifiziert, bis die mittlere Logphase erreicht ist. Dann werden die Zellen für 5 Minuten bei 1000 g abzentrifugiert und in Wasser resuspendiert und auf eine Zelldichte von 10⁷ pro Milliliter verdünnt.

Die so gewonnene Zellsuspension wird dann in einen fluoreszenzaktivierten Zellsorter vom Typ FX-Elite-ESP (Coulter) eingespeist und mit einer Zählrate von circa 20,000 Zellen pro Sekunde vermessen. Es wird mit einem 50mW UV-Laser im Bereich des Absorptionsmaximums von P4-3 bei 380nm angeregt und die Emission im Emissionsmaximum von S65T bei 511nm gemessen. Zellen, die den unspezifischen Hintergrund signifikant überschreiten, werden in Mikrotiterplatten mit leucinfreiem Minimalmedium gesammelt und durch Schütteln bei 30°C vermehrt. Die so isolierten Zellklone werden dann zur Isolierung der pGAD-GFP-Library Plasmide herangezogen, unter Verwendung der im Clontech-Handbuch beschriebenen Standardmethoden.

Zur Kontrolle werden die isolierten Klone dann in die Hefezellen zurücktransformiert und zwar einmal mit dem Köderplasmid pGBT-BFP-P2X2 und zum anderen mit dem leeren Köderplasmidvektor pGBT-BFP. Die Fluoreszenzspektren der beiden Transformanten werden dann verglichen, um solche Interaktionskandidaten auszuschließen, die direkt mit dem Blau Fluoreszierenden Protein, nicht aber mit dem Köderprotein interagieren.

So verifizierte Kandidaten werden dann wieder auf selektiven Minimalmediumplatten ausplattiert und die Sequenz der potentiell interagierenden Beutedomäne durch Standardmethoden der Molekularbiologie bestimmt.

### BEISPIEL 2

Zwei Vektoren werden verwendet, wobei der erste ausgewählt wird aus den kommerziell erhältlichen Expressionsvektoren für EBFP oder ECFP, z.B. pEBFP-N1, pEBFP-N2, pEBFP-N3, pEBFP-C1, pEBFP-C2, pEBFP-C3, pECFP, pECFP-Cl (Clontech) und der zweite aus den kommerziell erhältlichen Expressionsvektoren für EGFP oder EYFP, z.B. pEGFP-N1, pEGFP-N2, pEGFP-N3, pEGFP-C1, pEGFP-C2, pEGFP-C3, pEYFP-N1, pEYFP-N2, pEYFP-N3, pEYFP-C1, pEYFP-C2, pEYFP-C3.

In einen der beiden Vektoren wird wie üblich eine DNA-Sequenz kloniert, die ein Köderprotein kodiert, so daß das Köderprotein als Fusionsprotein mit dem fluoreszierenden Protein abgelesen wird. In den anderen wird eine cDNA-Bibliothek kloniert, oder eine kombinatorische Oligonukleotid-Bibliothek, so daß das zweite fluoreszierende Protein als Fusionsprotein mit jeweils einem Proteinfragment oder kombinatorischem Peptid abgelesen wird. Beide Vektoren zusammen werden dann in bekannter Weise in geeignete Säugerzellen, z.B. COS-7, NG-108, HIH/3T3 etc., transfiziert.

Nach einer ausreichenden Inkubationszeit werden die Zellen dann durch FRET-Mikroskopie analysiert (Siehe Clegg, In "Fluorescence Imaging Spectroscopy and Microscopy", S. 179-236, John Wiley & Sons, 1996) oder in oben beschriebener Weise durch fluoreszenzaktivierte Zellsortierung (FACS) vereinzelt. Die Zellen, in denen FRET auftritt, werden dann in der üblichen Weise, z.B. durch Isolierung des Cytoplasmas mit einer in der Elektrophysiologie gebräuchlichen Mikropipette und nachfolgender RT-PCR zur Ermittlung der zugrunde liegenden Beutesequenzen herangezogen.

## Patentansprüche

1. Verfahren zur Identifizierung von Peptid- bzw. Proteinwechselwirkungspartnern, bei welchem mindestens zwei Peptide bzw. Proteine an unterschiedlich fluoreszierende Komponenten gekoppelt sind, wobei die Absorptions- und Emissionsspektren der fluoreszierenden Komponenten derart überlappen, daß Fluoreszenz-Resonanz-Energie-Transfer möglich ist und die fluoreszierenden Komponenten durch eine Wechselwirkung zwischen den Proteinen bzw. Peptiden so zusammengebracht werden, daß FRET auftritt und gemessen wird, **dadurch gekennzeichnet, daß** die Peptide bzw. Proteine und fluoreszierenden Komponenten in Form von Fusionspeptiden bzw. -proteinen vorliegen und deren genetische Information in Wirtszellen in einem Expressionssystem eingebracht wird und FRET dort gemessen wird.

2. Verfahren nach Anspruch 1, wobei die fluoreszierenden Komponenten mit überlappenden Emissions- und Absorptionsspektren Blue Fluorescent Protein und Green Fluorescent Protein aus Aequorea victoria sind.

3. Verfahren nach Anspruch 1 oder Anspruch 2, wobei die Wirtszellen Hefezellen sind.

4. Verfahren nach einem der Ansprüche 1 bis 3, wobei die Messung des Fluoreszenz-Resonanz-Energie-Transfers mittels Fluoreszenzmikroskopie oder fluoreszenzgesteuerter Zellsortierung (FACS) erfolgt.

5. Verfahren nach einem der Ansprüche 1 bis 4, wobei die Anregung der Fluoreszenz mittels Laser geschieht.

6. Verfahren nach einem der Ansprüche 1 bis 5, wobei der Protein- bzw. Peptidanteil des Fusionsproteins bzw. -peptids aus einer kombinatorischen Peptidbibliothek stammt oder das Expressionsprodukt einer cDNA-Bibliothek ist.

7. Kit für die Identifizierung von Wechselwirkungen zwischen Proteinen bzw. Peptiden, umfassend
a) mindestens einen ersten Vektor mit
- einer kodierenden Sequenz für ein erstes fluoreszierendes Protein oder Peptid, und
- einer Klonierungsstelle, über die eine kodierende Sequenz für ein erstes Testprotein oder Testpeptid einbringbar ist,
und
b) mindestens einen zweiten Vektor mit
- einer kodierenden Sequenz für ein zweites fluoreszierendes Protein oder Peptid, dessen Absorptionsspektrum mit dem Emissionsspektrum des ersten fluoreszierenden Proteins oder Peptids derart überlappt, daß FRET auftritt, und
- einer Klonierungsstelle, über die eine kodierende Sequenz für ein zweites Testprotein oder Testpeptid einbringbar ist.

8. Kit nach Anspruch 7, **dadurch gekennzeichnet, daß** es sich bei den kodierenden Sequenzen für das erste und zweite fluoreszierende Protein oder Peptid um Sequenzen handelt, die für eine bestimmte Wirtszelle optimiert sind.

9. Kit nach Anspruch 7 oder Anspruch 8, **dadurch gekennzeichnet, daß** der erste Vektor und der zweite Vektor einen in einer bestimmten Wirtszelle funktionsfähigen Promotor umfaßt.

10. Kit nach einem der Ansprüche 7 bis 9, **dadurch gekennzeichnet, daß** der erste Vektor und/oder der zweite Vektor mindestens ein Markergen umfaßt, dessen Expression in der Wirtszelle eine Selektion der den jeweiligen Vektor enthaltenden Zellen erlaubt.

11. Kit nach einem der Ansprüche 7 bis 10, **dadurch gekennzeichnet, daß** die kodierende Sequenz für das erste Testprotein/Testpeptid und/oder die kodierende Sequenz für das zweite Testprotein/Testpeptid an der jeweiligen Klonierungsstelle bereits eingebracht ist.

12. Kit nach Anspruch 11, **dadurch gekennzeichnet, daß** es sich bei der kodierenden Sequenz um eine sogenannte Nukleotid-Bibliothek, vorzugsweise eine cDNA-Bibliothek handelt.

13. Kit nach einem der Ansprüche 7 bis 12, **dadurch gekennzeichnet, daß** es sich bei dem ersten Vektor und/oder dem zweiten Vektor um ein Plasmid handelt, wobei die entsprechende Klonierungsstelle vorzugsweise eine Restriktionserkennungsstelle ist.

14. Kit nach einem der Ansprüche 7 bis 13, **dadurch gekennzeichnet, daß** er zusätzlich eine Menge eines geeigneten Wirtszellentyps umfaßt, in dem der erste Vektor und/oder der zweite Vektor exprimierbar sind.

15. Kit nach Anspruch 14, daß es sich bei der Wirtszelle um eine Hefezelle handelt.

## Claims

1. Method for identifying peptide or protein interaction partners, in which at least two peptides or proteins are coupled to different fluorescent components, the absorption and emission spectra of the fluorescent components overlapping in such a way that fluorescence-resonance energy transfer is possible and the fluorescent components are so brought together by an interaction between the proteins or peptides that FRET occurs and is measured, **characterized in that** the peptides or proteins and fluorescent components are present in the form of fusion peptides or proteins and their genetic information is introduced into host cells in an expression system and FRET is measured there.

2. Method according to claim 1, wherein the fluorescent components with overlapping emission and absorption spectra are blue fluorescent protein and green fluorescent protein from Aequorea victoria.

3. Method according to claim 1 or 2, wherein the host cells are yeast cells.

4. Method according to one of the claims 1 to 3, wherein the measurement of the fluorescence-resonance energy transfer takes place by means of fluorescence microscopy or fluorescence-controlled cell sorting (FACS).

5. Method according to one of the claims 1 to 4, wherein fluorescence excitation takes place by laser.

6. Method according to one of the claims 1 to 5, wherein the protein or peptide fraction of the fusion protein or peptide comes from a combinatory peptide library or is the expression product of a cDNA library.

7. Kit for identifying interactions between proteins or peptides, comprising
a) at least one first vector with
- a coding sequence for a first fluorescent protein or peptide and
- a cloning site by means of which it is possible to introduce a coding sequence for a first test protein or test peptide and
b) at least one second vector with
- a coding sequence for a second fluorescent protein or peptide, whose absorption spectrum overlaps with the emission spectrum of the first fluorescent protein or peptide in such a way that FRET occurs and
- a cloning site by means of which one coding sequence for a second test protein or test peptide can be introduced.

8. Kit according to claim 7, **characterized in that** the coding sequences for the first and second fluorescent protein or peptide are sequences optimized for a specific host cell.

9. Kit according to claim 7 or 8, **characterized in that** the first vector and second vector comprise a promoter able to function in a specific host cell.

10. Kit according to one of the claims 7 to 9, **characterized in that** the first vector and/or the second vector has at least one marker gene, whose expression in the host cell permits a selection of the cells containing the particular vector.

11. Kit according to one of the claims 7 to 10, **characterized in that** the coding sequence for the first protein/test peptide and/or the coding sequence for the second test protein/test peptide is already introduced at the particular cloning site.

12. Kit according to claim 11, **characterized in that** the coding sequence is a so-called nucleotide library, preferably a cDNA library.

13. Kit according to one of the claims 7 to 12, **characterized in that** the first vector and/or the second vector is a plasmide, the corresponding cloning site preferably being a restriction detection site.

14. Kit according to one of the claims 7 to 13, **characterized in that** it additionally comprises a quantity of a suitable host cell type, in which the first vector and/or the second vector can be expressed.

15. Kit according to claim 14, **characterized in that** the host cell is a yeast cell.

## Revendications

1. Procédé pour l'identification de partenaires interactifs de peptides et de protéines, où au moins deux peptides respectivement deux protéines sont accouplés à différents composants fluorescents, les spectres d'absorption et d'émission des composants se chevauchant de sorte qu'un transfert énergie-résonance-fluorescence (TERF) soit possible et que les composants fluorescents soient rassemblés par une interaction entre les protéines et les peptides de manière que le TERF se produise et soit mesuré, **caractérisé en ce que** les peptides respectivement les protéines et les composants fluorescents existent sous forme de peptides ou protéines de fusion et dont l'information génétique est incorporée dans des cellules hôtes dans un système d'expression où est mesuré le TERF.

2. Procédé selon la revendication 1, **caractérisé en ce que** les composants fluorescents sont des protéines fluorescentes bleues avec des spectres d'absorption et d'émission superposés et des protéines fluorescentes vertes à partir d'Aequorea Victoria.

3. Procédé selon la revendication 1 ou la revendication 2, où les cellules hôtes sont des cellules de levure.

4. Procédé selon l'une des revendications 1 à 3, où la mesure du transfert énergie-résonance-fluorescence s'effectue au moyen de la microscopie fluorescente ou du tri des cellules commandé par fluorescence (FACS).

5. Procédé selon l'une des revendications 1 à 4, où l'amorce de la fluorescence s'effectue par laser.

6. Procédé selon l'une des revendications 1 à 5, où la partie en protéine et en peptide de la protéine de fusion respectivement de peptide de fusion est issue d'une bibliothèque de peptides combinatoire ou est le produit d'émission d'une bibliothèque cDNA.

7. Kit pour l'identification d'interactions entre les protéines et les peptides, comprenant
a) au moins un premier vecteur avec
- une séquence de codage pour une première protéine ou peptide fluorescente et
- un poste de clonage par lequel une séquence de codage peut être introduite pour une première protéine ou second peptide test,
et
b) au moins un second vecteur avec
- une séquence de codage pour une seconde protéine ou peptide fluorescente dont le spectre d'absorption est superposé au spectre d'émission de la première protéine ou peptide fluorescente de manière à ce que le TEFR se produise et
- un poste de clonage par lequel une séquence de codage peut être introduite pour une seconde protéine ou second peptide test.

8. Kit selon la revendication 1, **caractérisé en ce que** pour les séquences de codage pour la première et la seconde protéine ou le premier et le second peptide fluorescents, il s'agit de séquences qui sont optimisées pour une cellule hôte définie.

9. Kit selon la revendication 7 ou la revendication 8, **caractérisé en ce que** le premier vecteur et le second vecteur comprennent un promoteur actif dans une cellule hôte définie.

10. Kit selon l'une des revendications 7 à 9, **caractérisé en ce que** le premier vecteur et/ou le second vecteur comprennent au moins un gène marqueur dont l'expression dans la cellule hôte permet une sélection des cellules contenues dans le vecteur respectif

11. Kit selon l'une des revendications 7 à 10, **caractérisé en ce que** la séquence de codage pour la première protéine test ou premier peptide test et/ou la séquence de codage pour la seconde protéine test ou second peptide test sont introduites respectivement au niveau du poste de clonage respectif.

12. Kit selon la revendication 11, **caractérisé en ce qu'**il s'agit d'une bibliothèque dite nucléotide, de préférence une bibliothèque cDNA.

13. Kit selon l'une des revendications 7 à 12, **caractérisé en ce que** pour le premier vecteur et/ou le second vecteur, il s'agit d'un plasmide, le poste de clonage correspondant étant de préférence un point de détection de restriction.

14. Kit selon l'une des revendications 7 à 13, **caractérisé en ce qu'**il comprend en plus une quantité appropriée d'un type de cellules hôtes appropriées, type dans lequel le premier vecteur et/ou le second vecteur peuvent être exprimés.

15. Kit selon la revendication 14 **caractérisé en ce que** pour la cellule hôte, il s'agit d'une cellule de levure.
